# EUROPEAN PATENT SPECIFICATION

(11) **EP 2 536 412 B1**
(45) Date of publication and mention of the grant of the patent: **12.11.2014**
(21) Application number: 11703620.2
(22) Date of filing: 15.02.2011
(51) Int. Cl.: A61K 31/57, A61K 9/00

(54) **AN INHALABLE PHARMACEUTICAL COMPOSITION**
INHALIERBARE PHARMAZEUTISCHE ZUSAMMENSETZUNG
COMPOSITION PHARMACEUTIQUE INHALABLE

(30) Priority: 16.02.2010 US 304998 P
(43) Date of publication of application: 26.12.2012
(73) Proprietor: Teva Branded Pharmaceutical Products R & D, Inc., Horsham, PA 19044 (US)
(72) Inventor: DORINSKY, Paul Michael, Blue Bell, PA 19422 (US); PRICE, David, Sankence Aylsham NR11 6UN (GB)
(74) Representative: Cottam, David William
(86) International application number: PCT/EP2011/000692
(87) International publication number: WO 2011/101114

(56) References cited:
- US-A1- 2004 062 720
- US-A1- 2006 257 324
- TEVA SPECIALITY PHARMACEUTICALS: 'PRODUCT INFORMATION QVAR', [Online] 31 August 2008, XP055112944 Retrieved from the Internet: <URL:http://www.accessdata.fda.gov/drugsatf da_docs/label/2008/020911s017lbl.pdf> [retrieved on 2014-04-08]

## Description

### TECHNICAL FIELD OF THE INVENTION

This invention relates to an inhalable pharmaceutical composition, and particularly to an inhalable pharmaceutical solution aerosol comprising beclometasone dipropionate.

### BACKGROUND OF THE INVENTION

Asthma is a common inflammatory disease in which the airways of the respiratory system become chronically narrowed and constricted by oedema (fluid retention). During an attack, a patient suffers from laboured breathing accompanied especially by wheezing and coughing and by a sense of constriction in the chest due to bronchospasm (sudden constriction of the muscles in the walls of the bronchioles), mucosal oedema and mucus formation. Asthma is triggered by hyperreactivity to various stimuli (such as allergens or a rapid change in air temperature). In sensitised individuals, inhaled allergens (allergy triggers) provoke a hyperimmune response characterised by recruitment of immune cells and production of immunoglobulin E (IgE) antibodies. Asthma is caused by the attachment of IgE antibodies to mast cells (a resident cell of several types of tissues throughout the body, particularly in proximity to surfaces that interface the external environment). This attachment activates mast cells and on renewed exposure to the same antigen, degranulation of the mast cells occurs, leading to the rapid release of inflammatory mediators, such as histamine, proteoglycans, and cytokines. Asthma is a result of localised release of such mediators.

In a patient suffering from asthma, airflow obstruction is largely reversible and the patient experiences a significant response to inhaled bronchodilators and inhaled antiinflammatories.

Asthma is generally treated using a combination of long-term treatment and short-term episodic treatment of acute attacks. Long-term treatment of asthma involves the use of antiinflammatories and long-acting bronchodilators. Short-term episodic treatment employs short-acting bronchodilators. Inhaled glucocorticosteroids (ICS) are a feature of the currently preferred treatment for moderate to severe allergic asthma, and have been shown to act by suppressing the adaptive immune response while not suppressing innate immune response. There are various effective ICSs available in the art, for example, fluticasone propionate or CFC-beclometasone (a formulation comprising beclometasone dipropionate and a chlorofluorocarbon propellant).

Beclometasone dipropionate (INN), also known as beclomethasone dipropionate (USAN) or (8S, 9R, 10S, 11S, 13S, 14S, 16S, 17R)-9-chloro-11-hydroxy-10, 13, 16-trimethy)-3-oxo-17-[2-(propionyloxy)acetyl]-6,7,8,9,10,11,12,13,14,15,16,17-dodecahydro-3H-cyclopenta[a]phenanthren-17-yl propionate (IUPAC), is the subject of the present invention.

Formulations of beclometasone dipropionate are known in the art. For example, US 5,776,432 discloses a pharmaceutical solution aerosol formulation comprising beclometasone dipropionate, a hydrofluorocarbon propellant selected from 1 ,1 ,1,2-tetrafluoroethane (norflurane or propellant 134a), 1 , 1 ,1,2,3,3,3-heptafluoropropane (propellant 227) and a mixture thereof, and ethanol (anhydrous) to solubilise the beclometasone dipropionate in the propellant. The beclometasone dipropionate is dissolved in the formulation, and the formulation is substantially free of surfactant. However, further improvements in treatment are desired for the effective management of asthma.

US 2004/062720 discloses compositions which contain a corticosteroid, a propellant, a cosolvent, and an antioxidant for the treatment of bronchial disorders by administration via a pressurized metered dose inhaler. US 2006/0257324 discloses A method for delivering two or more active drug substances to the lungs by inhalation from a single pressurized metered dose inhaler product, the inhaler containing a HFA/co-solvent based solution formulation.

### SUMMARY OF THE INVENTION

Accordingly, the present invention provides an inhalable pharmaceutical solution aerosol comprising beclometasone dipropionate, ethanol and a propellant selected from 1,1,1,2-tetrafluoroethane, 1,1,1,2,3,3,3-heptafluoropropane and a mixture thereof, wherein the aerosol has a droplet size having a mass median aerodynamic diameter of 0.5-2.0 µm, for use in the treatment of asthma in a patient who has failed to maintain a lung function above 60% of baseline FEV₁ in response to inhalable fluticasone propionate and/or an inhalable formulation comprising beclometasone dipropionate and a chlorofluorocarbon propellant, by maintaining lung function above 60% of baseline FEV₁, or reducing the occurrences of lung function falling below 60% of baseline FEV₁.

This formulation provides a surprising improvement in the lung (pulmonary) function compared to other known ICSs.

### DETAILED DESCRIPTION OF THE INVENTION

The inhalable pharmaceutical solution aerosol of the present invention comprises beclometasone dipropionate, ethanol and a propellant selected from 1,1,1,2-tetrafluoroethane, 1,1,1,2,3,3,3-heptafluoropropane and a mixture thereof.

The active ingredient beclometasone dipropionate is generally present in a formulation of the invention in a therapeutically effective amount, i.e. an amount such that metered volumes of the medicament administered to the patient contains an amount of drug effective to exert the intended therapeutic action. The aerosol solution preferably contains 0.02 to 0.6 percent by weight, more preferably 0.05 to 0.5 percent by weight of beclometasone dipropionate, based on the total weight of the solution.

Ethanol is present in an amount effective to solubilise the beclometasone dipropionate in the propellant. Preferably, the solution contains 1 to 20 percent by weight of ethanol, more preferably 2 to 12 percent by weight and most preferably 4 to 10 percent by weight, based on the total weight of the aerosol solution. The ethanol will be present in an amount sufficient to dissolve substantially all of the medicament present in the formulation and to maintain the medicament dissolved over the time period and conditions experienced by commercial aerosol products. Preferably the ethanol is present in an amount to prevent precipitation of the active ingredient even at temperatures down to -20°C. The ethanol is preferably anhydrous ethanol, although trace amounts of water absorbed by the ingredients, for example during manufacture of the medicament, may be tolerated.

The hydrofluorocarbon propellant may be propellant 134a, propellant 227 or a mixture thereof. The solution preferably contains 80 to 99 percent by weight of propellant, more preferably 88 to 98 percent by weight, and most preferably 90 to 95 percent by weight, based on the total weight of the aerosol solution. The hydrofluorocarbon propellant is preferably the only propellant present in the formulations of the invention.

The solution of the present invention is preferably substantially free of surfactant. Surfactants are often added to suspensions to stabilise the suspension. However, since the formulation of the present invention is a solution, a surfactant is not required. Nevertheless, small quantities can be tolerated without adversely affecting the formulation. Preferably the formulation contains no more than 0.0005 percent by weight of a surfactant based on the total weight of the solution. Preferred formulations contain no surfactant. Presence of a significant amount of a surfactant is believed to be undesirable for solution formulations of beclometasone dipropionate because surfactants such as oleic acid and lecithin are believed to promote chemical degradation of the active ingredient when the latter is dissolved in the mixture of the propellant and ethanol.

A preferred solution according to the present invention comprises 0.02 to 0.6 percent by weight beclometasone dipropionate, 1 to 20 percent by weight ethanol and 80 to 99 percent by weight of propellant, wherein the percentages by weight are based on the total weight of the solution aerosol. A particularly preferred solution consists essentially of beclometasone dipropionate, ethanol and a propellant selected from 1,1,1,2-tetrafluoroethane, 1,1,1,2,3,3,3-heptafluoropropane and a mixture thereof; more preferably the solution consists of these components.

The solution of the present invention may be prepared by dissolving the desired amount of beclometasone dipropionate in the desired amount of ethanol accompanied by stirring or sonication. An aerosol container may then be filled using conventional cold-fill or pressure-fill methods.

The solution aerosol of the present invention is administered from a pressurised metered-dose inhaler (pMDI). A pMDI has two key components, namely a canister and an actuator (or mouthpiece). The canister has a vial for storing the solution coupled to a metering dose valve having an actuating stem. The container is housed in an actuator where the actuating step is in fluid communication with a discharge nozzle in the actuator. Actuation of the device releases a single metered dose of solution aerosol. The aerosol passes through the discharge nozzle resulting in a breaking up of the volatile propellant into droplets, followed by rapid evaporation of these droplets as they are inhaled into the lungs. The discharge nozzle preferably has an orifice diameter of 100-300 µm, more preferably 150-250 µm and most preferably 248 µm. This orifice size encourages the formation of droplets of the required size in the aerosol solution of the present invention.

The solution of the present invention when administered from the pMDI forms a fine aerosol of droplets which has a droplet size having a mass median aerodynamic diameter of 0.5-2.0 µm and preferably 0.8-1.2 µm. This small droplet size facilitates deep lung penetration. The droplet size may be measured using conventional techniques, such as using a Next Generation Pharmaceutical Impactor (NGI) or an Andersen eight-stage impactor (ACI).

It has been found that the solution aerosol of the present invention is capable of providing improved lung function and is able to maintain lung function above 60% of baseline FEV₁, or to reduce the occurrences of lung function falling below 60% of baseline FEV₁ in a patient suffering from asthma. Preferably the solution aerosol of the present invention may be used for maintaining lung function above 70% of baseline FEV₁, or reducing the occurrences of lung function falling below 70% of baseline FEV₁, and more preferably for maintaining lung function above 80% of baseline FEV₁, or reducing the occurrences of lung function falling below 80% of baseline FEV₁. Reducing the occurrences is compared to a patient taking no ICS or even other ICS, such as fluticasone propionate and/or CFC-beclometasone dipropionate.

FEV₁ is the maximal volume of air exhaled in the first second of a forced expiration from a position of full inspiration, expressed in litres at body temperature and ambient pressure saturated with water vapour (BTPS). FEV₁ is determined by spirometry which is the most common of the Pulmonary Function Tests (PFTs). FEV₁ is measured according to the parameters set down in the joint statements on lung function testing by the American Thoracic Society (ATS) and the European Respiratory Society (ERS), see the Series "ATS/ERS Task Force: Standardisation of Lung Function Testing, Edited by V. Brusasco, R. Crapo and G. Viegi, Numbers 1-5, Eur. Respir. J. 2005; 26: 153-161, 319-338, 511-522, 720-735 and 948-968.

The FEV₁ obtained is expressed in litres for an individual patient. It must then be compared to a reference (predicted) value based on healthy subjects. Predicted values are obtained from studies of normal, healthy subjects with the same anthropometric (sex, age and height) and ethnic characteristics of the patient being tested. Reference values are derived from large groups of volunteers. The criteria to define subjects as normal or healthy have been laid down the ATS and ERS.

The result is an FEV₁ expressed as a percentage of predicted. The severity of the asthma is defined by this percentage. The ATS/ERS statements lay down the following definitions: mild asthma is an FEV₁ of 70% of predicted or above; moderate 60-69% of predicted; moderately severe 50-59% of predicted; severe 35-49% of predicted and very severe less than 35% of predicted The percentage provides a baseline measurement for any given patient. It has been found that the solution aerosol of the present invention maintains the baseline for a given patient within the above-described limits.

A group of patients has also been identified which receive particular benefit from the solution aerosol of the present invention. That is, a patient showing insufficient response to inhalable fluticasone propionate and/or an inhalable formulation comprising beclometasone dipropionate and a chlorofluorocarbon propellant. An insufficient response herein is defined as failing to maintain the baseline for a given patient within the above-described limits.

The present invention also provides a method for maintaining lung function above 60% of baseline FEV₁, or reducing the occurrences of lung function falling below 60% of baseline FEV₁ by administering to a patient in need thereof, an inhalable pharmaceutical solution aerosol comprising beclometasone dipropionate, ethanol and a propellant selected from 1,1,1,2-tetrafluoroethane, 1,1,1,2,3,3,3-heptafluoropropane and a mixture thereof, wherein the aerosol has a droplet size having a mass median aerodynamic diameter of 0.5-2.0 µm.

The present invention will now be described with reference to the following example, which is not intended to be limiting.

### EXAMPLE

Trials were conducted using Qvar® which is a solution aerosol formulation containing beclometasone dipropionate, anhydrous ethanol, propellant 134a. Qvar is available in two doses, 50 µg and 100 µg. The two formulations contain: beclometasone dipropionate 0.05 or 0.10 mg per actuation, anhydrous ethanol 4.74 mg per actuation and propellant 134a 54.51 or 54.46 mg per actuation. Both formations have a density at 20°C of 1.166 g/mL and a MMAD of 1.1 µm.

Qvar and other inhaled corticosteroids (ICS) CFC-beclometasone (BDP) and fluticasone propionate (FP) were compared Qvar in a large representative, UK clinical database.

Asthma patients, aged 5-60, without other chronic respiratory diseases, receiving Qvar, CFC-BDP or FP MDIs who subsequently had ICS increase between January 1997-June 2006 were identified from UK General Practice Research Database. Multiple logistic regression, adjusted for baseline severity provided odds of successful asthma control (no asthma hospital attendances, oral steroids, consultations or hospital admissions for lower respiratory tract infections requiring antibiotics) during 12 month follow-up. Poisson regression was used to compare asthma exacerbation rates (unscheduled hospital admissions/ A&E attendances for asthma or use of oral steroids).

Data were available for 4133 patients (8% Qvar, 15% FP, 77% BDP). Odds ratio (95% CI) for success was lower with BDP and FP compared with Qvar (0.65 (0.47-0.90) and 0.71 (0.50-1.02) respectively. Exacerbation rates (95% CI) were higher with BDP and FP 1.44 (0.94-2.21) and 1.61 (1.02-2.55), respectively.

Patients receiving increased ICS therapy with Qvar were more likely to achieve successful asthma control.

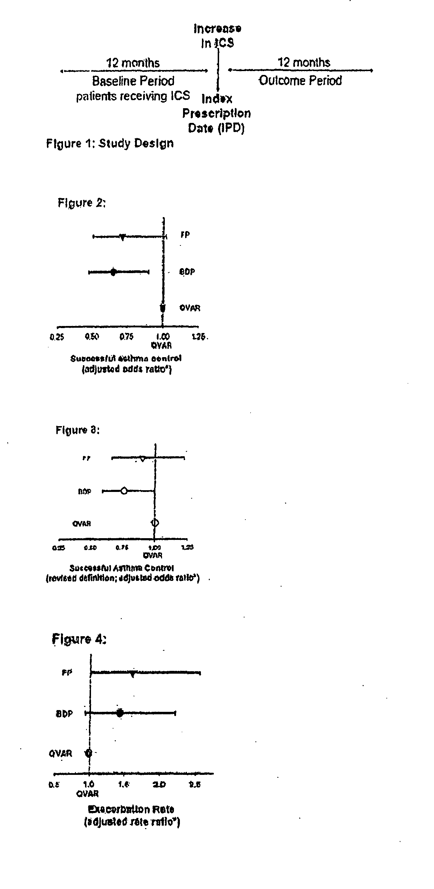

## Claims

1. An inhalable pharmaceutical solution aerosol comprising beclometasone dipropionate, ethanol and a propellant selected from 1,1,1,2-tetrafluoroethane, 1,1,1,2,3,3,3-heptafluoropropane and a mixture thereof, wherein the aerosol has a droplet size having a mass median aerodynamic diameter of 0.5-2.0 µm, for use in the treatment of asthma in a patient who has failed to maintain a lung function above 60% of baseline FEV₁ in response to inhalable fluticasone propionate and/or an inhalable formulation comprising beclometasone dipropionate and a chlorofluorocarbon propellant, by maintaining lung function above 60% of baseline FEV₁, or reducing the occurrences of lung function falling below 60% of baseline FEV₁.

2. An inhalable pharmaceutical solution aerosol for use as claimed in claim 1, comprising 0.02 to 0.6 percent by weight beclometasone dipropionate, 1 to 20 percent by weight ethanol and 80 to 99 percent by weight of propellant, wherein the percentages by weight are based on the total weight of the solution aerosol.

3. An inhalable pharmaceutical solution aerosol for use as claimed in claim 1, consisting essentially of beclometasone dipropionate, ethanol and a propellant selected from 1,1,1,2-tetrafluoroethane, 1,1,1,2,3,3,3-heptafluoropropane and a mixture thereof.

4. An inhalable pharmaceutical solution aerosol for use as claimed in claim 1, wherein the aerosol has a droplet size having a mass median aerodynamic diameter of 0.8-1.2 µm.

5. An inhalable pharmaceutical solution aerosol for use as claimed in claim 1, for maintaining lung function above 70% of baseline FEV₁, or reducing the occurrences of lung function falling below 70% of baseline FEV₁.

## Patentansprüche

1. Ein pharmazeutisches Lösungsraerosol zum Inhalieren, das Beclometason-Dipropionat, Ethanol und ein Treibmittel enthält, das aus 1,1,1 ,2-Tetrafluorethan, 1,1,1,2,3,3,3-Heptafluorpropan und einer Mischung aus diesen gewählt wird, wobei das Aerosol eine Tropfengröße mit einem massenbezogenen medianen aerodynamischen Durchmesser von 0,5-2,0 µm hat, zur Verwendung bei der Behandlung von Asthma bei einem Patienten, bei dem bei Gabe von inhalierbarem Fluticasonpropionat und/oder einer Inhallerbaren Rezeptur aus Beclometason-Dipropionat und einem Chlorfluorkohlenwasserstoff-Treibmittel eine Lungenfunktion über 60 % des Baseline-Ausgangswerts FEV₁ nicht aufrecht erhalten werden konnte, indem die Lungenfunktion über 60 % des Baseline-Ausgangswerts FEV₁ aufrecht erhalten wird oder die Reduzierung der Häufigkeit einer unterhalb von 60 % des Baseline-Ausgangswerts FEV₁ liegenden Lungenfunktion erreicht wird.

2. Ein pharmazeutisches Lösungsraerosol zum Inhalieren zur Verwendung nach Anspruch 1, das 0,02 bis 0,6 Massenanteil Beclometason-Dipropionat, 1 bis 20 Massenanteil Ethanol und 80 bis 99 Massenanteil Treibmittel enthält, wobei sich die Massenprozente auf die Gesamtmasse des Lösungsraerosols beziehen.

3. Ein pharmazeutisches Lösungsraerosol zum Inhalieren zur Verwendung nach Anspruch 1. das im Wesentlichen aus Beclometason-Dipropionat, Ethanol und einem Treibmittel besteht, das aus 1,1,1,2-Tetrafluorethan. 1,1,1,2,3,3.3-Heptafluorpropan und einer Mischung aus diesen gewählt wird.

4. Ein pharmazeutisches Lösungsraerosol zum Inhalieren zur Verwendung nach Anspruch 1, wobei das Aerosol eine Tropfengröße mit einem massenbezogenen medianen aerodynamischen Durchmesser von 0,8-1,2 µm hat.

5. Ein pharmazeutisches Lösungsraerosol zum Inhalieren zur Verwendung nach Anspruch 1 zur Aufrechterhaltung der Lungenfunktion über 70 % des Baseline-Ausgangswerts FEV₁ oder zur Reduzierung der Häufigkeit einer unterhalb von 70 % des Baseline-Ausgangswerts FEV₁ liegenden Lungenfunktion.

## Revendications

1. Une composition d'aérosol en solution pharmaceutique inhalable comprenant du dipropionate de béclométasone, de l'alcool éthylique et un agent de propulsion choisi entre le 1,1,1,2-tétrafluoroéthane, le 1,1,1, 2,3, 3, 3-heptafluoropropane et un mélange de ces agents, dans laquelle la taille des gouttelettes de l'aérosol présente un diamètre aérodynamique moyen en masse de 0,5 à 2,0 µg, utilisée pour le traitement de l'asthme chez les patients pour lesquels il a été impossible de préserver une fonction pulmonaire supérieure à 60 % du VEF₁ (volume expiratoire forcé) de référence en réponse au propionate defluticasone inhalable et/ ou une formulation inhalable comprenant du dipropionate de béclométasone et un agent de propulsion chlorofluorocarboné, permettant de préserver une fonction pulmonaire supérieure à 60 % du VEF₁ de référence, ou de réduire le nombre de fois où la fonction pulmonaire chute à un niveau inférieur à 60 % du VEF₁ de référence,

2. Une composition d'aérosol en solution pharmaceutique inhalable utilisée selon la revendication 1, comprenant 0,02 à 0,6 pourcents, en poids, de dipropionate de béclométasone, 1 à 20 pourcents, en poids, d'alcool éthylique et 80 à 99 pourcents, en poids, d'agent propulseur, dans laquelle les pourcentages en poids sont basés sur le poids total de la solution aérosol.

3. Une composition d'aérosol en solution pharmaceutique inhalable utilisée selon la revendication 1 comprenant essentiellement du dipropionate de béclamétasone, de l'alcool éthylique et un agent de propulsion choisi entre le 1,1,1,2-tétrafluoroéthane, le 1,1,1,2, 3,3,3-heptafluoropropane et un mélange de ces agents.

4. Une composition d'aérosol en solution pharmaceutique inhalable utilisée selon la revendication 1, dans laquelle la taille des gouttelettes de l'aérosol présente un diamètre aérodynamique moyen en masse de 0,8 à 1,2 µg.

5. Une composition d'aérosol en solution pharmaceutique inhalable utilisée selon la revendication 1, permettant de préserver une fonction pulmonaire supérieure à 70 % du VEF₁ de référence, ou de réduire le nombre de fois où la fonction pulmonaire chute à un niveau inférieur à 70 % du VEF₁ de référence.
